# EUROPÄISCHE PATENTSCHRIFT

(11) **EP 0 524 985 B1**
(45) Veröffentlichungstag und Bekanntmachung des Hinweises auf die Patenterteilung: **19.07.1995**
(21) Anmeldenummer: 91907141.5
(22) Anmeldetag: 05.04.1991
(51) Int. Cl.: C07C 59/125, C07C 51/42, B01D 29/35

(54) **VERFAHREN ZUR KATALYSATORRÜCKGEWINNUNG**
PROCESS FOR CATALYST RECOVERY
PROCEDE DE RECUPERATION D'UN CATALYSEUR

(30) Priorität: 14.04.1990 DE 4012128
(43) Veröffentlichungstag der Anmeldung: 03.02.1993
(73) Patentinhaber: HOECHST AKTIENGESELLSCHAFT, 65926 Frankfurt am Main (DE)
(72) Erfinder: LEUPOLD, Ernst, Ingo, D-6392 Neu-Anspach (DE); KAUFMANN, Wolf-Ditmar, D-6242 Kronberg (DE); LAUGWITZ, Bernd, D-6233 Kelkheim (DE); DUCHATSCH, Günther, D-6237 Liederbach (DE); MEYER-BLUMENROTH, Ulrich, D-6270 Idstein (DE)
(86) Internationale Anmeldenummer: EP9100649
(87) Internationale Veröffentlichungsnummer: WO9116294

(56) Entgegenhaltungen:
- DE-A- 2 936 123
- US-A- 2 757 800
- US-A- 4 861 471
- Ullmanns Encyclopedia of Industrial Chemistry Edition 5, 1988, Band B2, VCH Verlagsgesellschaft (Weinheim, DE) siehe Seiten 10-54

## Beschreibung

Die vorliegende Erfindung betrifft ein Verfahren zur Katalysatorrückgewinnung bei der Herstellung von Ethercarbonsäuren durch katalytische Oxidation der entsprechenden Etheralkohole mit Sauerstoff an suspendierten Edelmetall-Katalysatoren.

Die katalytische Oxidation von Etheralkoholen gemäß der allgemeinen Gleichung 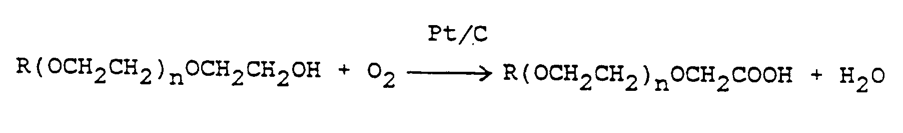
ist seit längerem bekannt und beispielsweise in DE-PS 29 36 123 und EP-PS 206 054 beschrieben. Mit zunehmender Molmasse des Restes R wird jedoch die Abtrennung und damit die vollständige Wiedergewinnung und Rückführung des Edelmetallkatalysators problematisch. So wird in der DE-PS 34 46 561 auf Seite 4 eine aufwendige vierstufige Methode beschrieben, um die Verluste an Edelmetall zu begrenzen. Ein wesentlicher verfahrenstechnischer Nachteil dieser Arbeitsweise besteht darin, daß das Reaktionsgemisch vor der Filtration mit der 1-10-fachen Menge an Aceton verdünnt wird, das anschließend wieder destillativ abgetrennt, gereinigt und zurückgeführt werden muß. Auch die schließlich erreichbaren Edelmetallgehalte im Produkt von 1-4 ppm sind relativ hoch und führen zur allmählichen Aktivitätsabnahme des Katalysators. Insgesamt ist damit ein wirtschaftlich schwerwiegender Nachteil verbunden.

Es bestand daher die Aufgabe, ein technisch und wirtschaftlich tragbares Verfahren zur Katalysatorrückgewinnung zu entwickeln.

Gegenstand der vorliegenden Erfindung ist nun ein Verfahren zur Katalysatorrückgewinnung bei der Herstellung von Ethercarbonsäuren durch katalytische Oxidation mit suspendiertem Katalysator, dadurch gekennzeichnet, daß man das Reaktionsgemisch einer Querstrom-Filtration unterwirft.

Bei den Ethercarbonsäuren handelt es sich vorzugsweise um solche der allgemeinen Formel

R-(OCH₂CH₂)ₙOCH₂COOH

in welcher R einen linearen oder verzweigten Alkylrest von 1 bis 24 Kohlenstoffatomen, einen Arylrest, wie beispielsweise einen Phenyl-, Naphthyl- oder Biphenylrest, ferner einen Alkyl(C₁-C₂₄)-arylrest, wobei der Arylrest beispielsweise einen Phenyl-, Naphthyl- oder Biphenylrest darstellt, oder einen Arylalkylrest, beispielsweise einen Benzylrest, und n eine Zahl von 0 bis 24 bedeuten. Die vorstehend genannten Arylreste können substituiert sein.

Bei der Querstrom-Filtration wird das katalysatorhaltige Reaktionsgemisch mit hoher Überströmgeschwindigkeit tangential zur Membranoberfläche durch das Filterelement gepumpt, wobei das Filtrat quer zur Strömungsrichtung durch die Membranschicht abgeführt wird, wie es im einzelnen beschrieben wird in ULLMANN's Encyclopedia of Industrial Chemistry, 5th Edition, Vol. B2, p. 10-54.

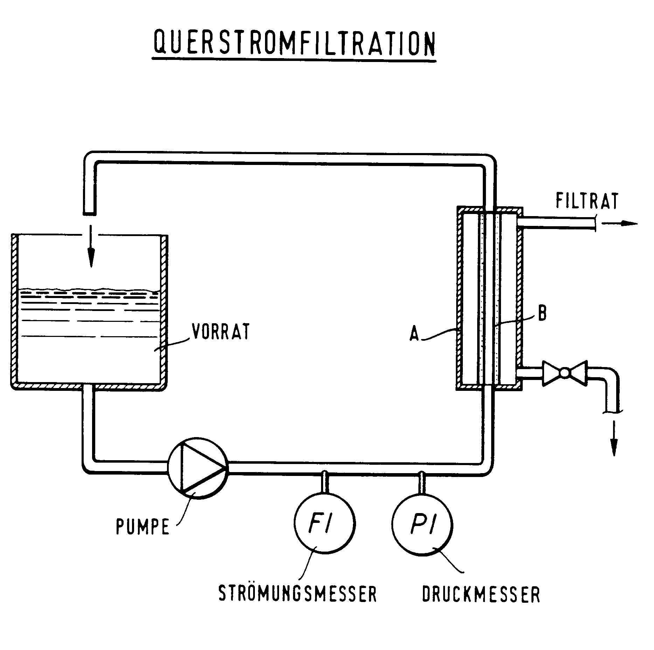

Besonders geeignet sind röhrenförmige Filterelemente mit einer Membranschicht auf der Innenseite der Röhren. Bevorzugtes Membran- und Trägermaterial ist α-Al₂O₃ und ZrO₂; aber auch Kunststoff- und Kohlenstoffelemente können eingesetzt werden. Membran und Träger können aus verschiedenen Materialien bestehen. Die notwendigen Porengrößen liegen zweckmäßig in Ultrafiltrationsbereich, beispielsweise zwischen etwa 1 und etwa 200 nm. Eine geeignete Apparatur ist im Beispiel beschrieben (vgl. hierzu auch Figur 1 auf Seite 8).

Die erfindungsgemäße Querstrom-Filtration kann bei Temperaturen von 20 bis 150°C vorgenommen werden. Als vorteilhaft hat sich erwiesen, die Filtration bei Temperaturen von 50 bis 100°C, insbesondere bei Temperaturen von 60 bis 80°C, durchzuführen.

Es versteht sich von selbst, daß zwischen Vorder- und Rückseite der Membran ein Druckgefälle herrschen muß, damit die Reaktionslösung hindurchbewegt werden kann.

Ein bei der Oxidation verwendeter Lösevermittler kann die Filtration erleichtern. Geeignet sind Lösevermittler ohne Hydroxylgruppen. Besonders geeignet sind Glykolether ohne Hydroxygruppen, insbesondere Diethylenglykoldimethylether.

Eine kurzzeitige Wasserstoffbehandlung nach dem Ende der Oxidation bei Reaktionstemperatur ( 50 bis 100°C) hat sich als vorteilhaft erwiesen, um eventuell gelöste und kolloidale Edelmetallspuren abzuscheiden, wodurch Filterelemente mit etwas größeren Poren verwendbar werden können. Als Reduktionsmittel kann außer Wasserstoff beispielsweise auch Formaldehyd eingesetzt werden.

Überraschenderweise werden nach den erfindungsgemäßen Verfahren die Probleme der Filtration (Verstopfung, Edelmetallverluste) gelöst, ohne daß eine wirtschaftlich aufwendige Maßnahme, wie beispielsweise das Verdünnen mit einem Lösemittel, erforderlich ist.

Das nachstehende Beispiel dient zur Erläuterung des erfindungsgemäßen Verfahrens, ohne es darauf zu beschränken.

### Beispiel

Eine Reaktionslösung aus der katalytischen Oxidation, bestehend aus 25 Gew. -% Ethercarbonsäuren der Formel R-(OCH₂CH₂)ₙOCH₂COOH, mit linearen Alkylgruppen R mit einer Verteilung von C₁₂ bis C₁₄ und einem Mittelwert n = 4 sowie 45 Gew.-% Diethylenglykoldimethylether, 25 Gew.-% Wasser und 5 Gew.-% eines suspendierten handelsüblichen Katalysators (5 Gew.-% Platin auf Aktivkohle) wird 30 Minuten bei 70-°C mit Wasserstoff in einer Blasensäule behandelt und anschließend einer Querstromfiltration unterworfen.

Das Filterelement besteht aus einem ZrO₂-Rohr (Durchmesser: 7 mm Länge: 250 mm), dessen Innenseite aus einer Membranschicht mit Porengrößen von 35 nm (10⁻⁹ m) besteht. Es wird eine Apparatur verwendet, welche der beigefügten Zeichnung (vgl. Figur 1 auf Seite 8) entspricht. Die Reaktionslösung wird mit einer linearen Strömungsgeschwindigkeit von 5 m/s durch das in einem Gehäuse (A) befindlichen Filterelement (B) gepumpt. Es wird ein Druck P₁ von 3 bar bei einer Temperatur von 70°C eingestellt. Man erhält einen Filtratstrom von 2,5 l/h. Die Aufkonzentrierung des Katalysators gelingt bis zu einem Feststoffanteil von ca. 30 Gew.-%; dieses Konzentrat wird in die katalytische Oxidation zurückgeführt.

Nach destillativer Abtrennung von Diglykoldimethylether und Wasser aus dem Filtrat erhält man das Gemisch der Ethercarbonsäuren als klares, helles Produkt mit einer Restmenge an Platin von unter 0,5 ppm.

### Vergleichsbeispiel

Die im vorstehenden Beispiel beschriebene Reaktionslösung wird mehrfach über eine Nutsche mit eingelegtem Filterpapier (für quantitative Analyse) filtriert. Wegen Verstopfens muß das Filterpapier öfters gewechselt werden. Man erhält ein trübes Filtrat, das nach Aufarbeitung gemäß Beispiel ein dunkles, trübes Produkt mit einem Platin-Gehalt von 36 ppm ergibt.

## Patentansprüche

1. Verfahren zur Katalysatorrückgewinnung bei der Herstellung von Ethercarbonsäuren durch katalytische Oxidation mit suspendiertem Katalysator, dadurch gekennzeichnet, daß man das Reaktionsgemisch einer Querstrom-Filtration unterwirft.

2. Verfahren nach Anspruch 1, dadurch gekennzeichnet, daß es sich bei den Ethercarbonsäuren um solche der allgemeinen Formel
R-(OCH₂CH₂)ₙOCH₂COOH
in welcher R einen linearen oder verzweigten Alkylrest mit 1 bis 24 Kohlenstoffatomen, einen am aromatischen Kern ggf. substituierten Aryl-, Alkylaryl- oder Arylalkylrest und n eine Zahl von 0 bis 24 bedeuten , handelt.

3. Verfahren nach mindestens einem der Ansprüche 1 und 2, dadurch gekennzeichnet, daß das Filterelement bei der Querstrom-Filtration aus keramischem Material und/oder Kohlenstoff besteht.

4. Verfahren nach mindestens einem der Ansprüche 1 bis 3, dadurch gekennzeichnet, daß das Filterelement bei der Querstrom-Filtration aus ZrO₂ und/oder α-Al₂O₃ besteht.

5. Verfahren nach mindestens einem der Ansprüche 1 bis 4, dadurch gekennzeichnet, daß man die Filtration bei Temperaturen von 20 bis 150°C durchführt.

6. Verfahren nach mindestens einem der Ansprüche 1 bis 5, dadurch gekennzeichnet, daß man die Filtration bei Temperaturen von 50 bis 100°C durchführt.

7. Verfahren nach mindestens einem der Ansprüche 1 bis 6, dadurch gekennzeichnet, daß man die Filtration bei Temperaturen von 60 bis 80°C durchführt.

8. Verfahren nach mindestens einem der Ansprüche 1 bis 7, dadurch gekennzeichnet, daß man die Querstrom-Filtration in Gegenwart eines Lösevermittlers ohne Hydroxylgruppen vornimmt.

9. Verfahren nach mindestens einem der Ansprüche 1 bis 8, dadurch gekennzeichnet, daß man die Querstrom-Filtration in Gegenwart eines Glykolethers ohne Hydroxylgruppen vornimmt.

10. Verfahren nach mindestens einem der Ansprüche 1 bis 9, dadurch gekennzeichnet, daß man die Querstrom-Filtration in Gegenwart von Diethylenglykoldimethylether vornimmt.

11. Verfahren nach mindestens einem der Ansprüche 1 bis 10, dadurch gekennzeichnet, daß man das Reaktionsgemisch vor der Querstrom-Filtration mit Wasserstoff bei Temperaturen von 50 bis 100°C behandelt.

12. Verfahren nach mindestens einem der Ansprüche 1 bis 10, dadurch gekennzeichnet, daß man das Reaktionsgemisch mit einem Reduktionsmittel, vorzugsweise mit Wasserstoff oder Formaldehyd behandelt.

## Claims

1. A process for the recovery of catalyst in the preparation of ether-carboxylic acids by catalytic oxidation using a suspended catalyst, which comprises the reaction mixture being subjected to a cross-flow filtration.

2. The process as claimed in claim 1, wherein the ether-carboxylic acids are those of the formula
R-(OCH₂CH₂)ₙOCH₂COOH
in which R is a linear or branched alkyl radical having 1 to 24 carbon atoms, an aryl, alkylaryl or arylalkyl radical unsubstituted or substituted at the aromatic nucleus and n is a number from 0 to 24.

3. The process as claimed in at least one of claims 1 and 2, wherein the filter element in the cross-flow filtration is composed of ceramic material and/or carbon.

4. The process as claimed in at least one of claims 1 to 3, wherein the filter element in the cross-flow filtration is composed of ZrO₂ and/or α-Al₂O₃.

5. The process as claimed in at least one of claims 1 to 4, wherein the filtration is carried out at temperatures from 20 to 150°C.

6. The process as claimed in at least one of claims 1 to 5, wherein the filtration is carried out at temperatures from 50 to 100°C.

7. The process as claimed in at least one of claims 1 to 6, wherein the filtration is carried out at temperatures from 60 to 80°C.

8. The process as claimed in at least one of claims 1 to 7, wherein the cross-flow filtration is performed in the presence of a solubilizer without hydroxyl groups.

9. The process as claimed in at least one of claims 1 to 8, wherein the cross-flow filtration is performed in the presence of a glycol ether without hydroxyl groups.

10. The process as claimed in at least one of claims 1 to 9, wherein the cross-flow filtration is performed in the presence of diethylene glycol dimethyl ether.

11. The process as claimed in at least one of claims 1 to 10, wherein the reaction mixture, prior to the cross-flow filtration, is treated with hydrogen at temperatures from 50 to 100°C.

12. The process as claimed in at least one of claims 1 to 10, wherein the reaction mixture is treated with a reducing agent, preferably with hydrogen or formaldehyde.

## Revendications

1. Procédé de récupération de catalyseur lors de la préparation d'éthers-acides carboxyliques par oxydation catalytique avec un catalyseur mis en suspension, caractérisé en ce qu'on soumet le mélange réactionnel à une filtration tangentielle dite "à angle droit" ("Querstrom")

2. Procédé selon la revendication 1, caractérisé en ce qu'il s'agit, en ce qui concerne les éthers-acides carboxyliques, de ceux de formule générale
R-(OCH₂CH₂) ₙOCH₂COOH
dans laquelle R signifie un reste alkyle linéaire ou ramifié ayant de 1 à 24 atomes de carbone, un reste arylalkyle, un reste alkylaryle, un reste aryle, dont le noyau aromatique est éventuellement substitué, et n signifie un nombre compris entre 0 et 24.

3. Procédé selon au moins l'une des revendications 1 à 2, caractérisé en ce que l'élément de filtration lors de la filtration tangentielle est formé d'un matériau céramique et/ou de carbone.

4. Procédé selon au moins l'une des revendications 1 à 3, caractérisé en ce que l'élément de filtration lors de la filtration tangentielle est formé de ZrO₂ et/ou de α-Al₂O₃.

5. Procédé selon au moins l'une des revendications 1 à 4, caractérisé en ce qu'on réalise la filtration a des températures comprises entre 20 et 150°C.

6. Procédé selon au moins l'une des revendications 1 à 5, caractérisé en ce qu'on réalise la filtration à des températures comprises entre 50 et 100°C.

7. Procédé selon au moins l'une des revendications 1 à 6, caractérisé en ce qu'on réalise la filtration à des températures comprises entre 60 et 80°C.

8. Procédé selon au moins l'une des revendications 1 à 7, caractérisé en ce qu'on effectue la filtration tangentielle en présence d'un solvant n'ayant pas de groupe hydroxyle.

9. Procédé selon au moins l'une des revendications 1 à 8, caractérisé en ce qu'on effectue la filtration tangentielle en présence d'un glycoléther n'ayant pas de groupe hydroxyle.

10. Procédé selon au moins l'une des revendications 1 à 9, caractérisé en ce qu'on effectue la filtration tangentielle en présence de diéthylèneglycol-diméthyl-éther.

11. Procédé selon au moins l'une des revendications 1 à 10, caractérisé en ce qu'on traite le mélange réactionnel avant la filtration tangentielle avec de l'hydrogène à des températures comprises entre 50 et 100°C.

12. Procédé selon au moins l'une des revendications 1 à 10, caractérisé en ce qu'on traite le mélange réactionnel avec un réducteur, de préférence avec de l'hydrogène ou du formaldéhyde.
